(19) Europäisches Patentamt
European Patent Office
Office européen des brevets

(11) Publication number: **0 195 597**
**B1**

(12) # EUROPEAN PATENT SPECIFICATION

(45) Date of publication of patent specification: **02.11.89**

(51) Int. Cl.$^4$: **C 11 D 3/39,** C 07 C 179/10

(21) Application number: **86301804.0**

(22) Date of filing: **13.03.86**

(54) **Bleach activator compounds and bleaching compositions containing them.**

(30) Priority: **14.03.85 US 711901**

(43) Date of publication of application:
**24.09.86 Bulletin 86/39**

(45) Publication of the grant of the patent:
**02.11.89 Bulletin 89/44**

(84) Designated Contracting States:
**BE DE FR GB IT NL**

(56) References cited:
**EP-A-0 106 584**
**US-A-4 483 781**
**US-A-4 486 327**

(73) Proprietor: **THE PROCTER & GAMBLE COMPANY**
**One Procter & Gamble Plaza**
**Cincinnati Ohio 45202 (US)**

(72) Inventor: **Burns, Michael Eugene**
**9248 Sunderland Way**
**West Chester Ohio 45069 (US)**

(74) Representative: **Brooks, Maxim Courtney et al**
**Procter & Gamble (NTC) Limited Whitley Road**
**Longbenton**
**Newcastle-upon-Tyne NE12 9TS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 195 597 B1

**Description**

This invention relates to bleach activator compounds and peroxygen bleaching compositions that provide effective surface bleaching of textiles over a wide range of temperatures. Surface bleaching of textiles is bleaching wherein the bleaching mechanism takes place on the textile surface, and, thereby, removes stains and/or soils.

The bleach activator compounds of the invention provide effective and efficient surface bleaching of textiles which thereby removes stains and/or soils from the textiles. The compounds are particularly efficient at removing dingy soils from textiles. Dingy soils are those that build up on textiles after much usage and washing, and result in a gray or yellow tint on a white textile. These soils are a blend of particulate and greasy materials.

The compounds of the invention provide effective bleaching over a wide range of temperature (5°C to 85°C), a preferred range being from 30°C to 60°C.

The compounds of the invention perform surprisingly well under full soil load conditions. Alkyl substituted aromatic peroxyacids with comparable chain length substitution do not provide effective bleaching in the presence of soil.

US—A—4,154,695, McCrudden et al, issued May 15, 1979, and US—A—4,288,388, McCrudden et al, issued September 8, 1981, disclose aromatic peroxyacids for use in bleaching compositions. They specifically disclose aromatic peroxyacids with a further substitution of the benzene ring. Lower alkyl groups are described as a possible substitution, methyl being the only group specifically mentioned.

Surprisingly, bleach activator compounds which generate $C_4$—$C_8$ alkoxy substituted aromatic peroxyacids provide highly effective bleaching even in the presence of high soil loads, such as those found in many typical consumer wash loads.

The bleach activators within the invention are of the general formula

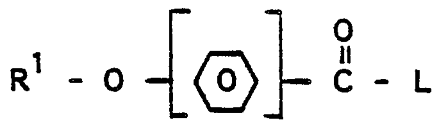

wherein $R^1$ is an alkyl group containing from 4 to 8 carbon atoms and L is a leaving group, the conjugate acid of which has a pKa in the range of from 4 to 13. A leaving group is any group that is displaced from the bleach activator as a consequence of the nucleophilic attack on the bleach activator by the perhydroxide anion. This, the perhydrolysis reaction, results in the formation of the corresponding peroxycarboxylic acid. Generally, for a group to be a suitable leaving group it must exert an electron attracting effect. It should also form a stable entity so that the rate of the back reaction is negligible. This facilitates the nucleophilic attack by the perhydroxide anion.

The L group must be sufficiently reactive for the reaction to occur within the optimum time frame (e.g., a wash cycle). However, if L is too reactive, this activator will be difficult to stabilize for use in a bleaching or detergent composition.

These characteristics are generally paralleled by the pKa of the conjugate acid of the leaving group, although exceptions to this convention are known. Leaving groups that exhibit such behavior are those in which their conjugate acid has a pKa in the range of from 4 to 13, preferably from 6 to 11 and most preferably from 8 to 11.

Preferred bleach activators are those of the above general formula wherein $R^1$ is as defined above and L is selected from the group consisting of:

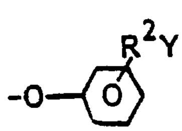 , 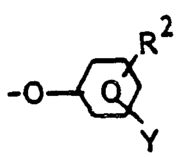 , 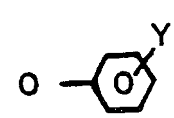 , 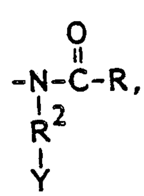

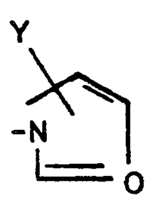 , 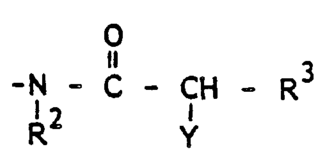 ,

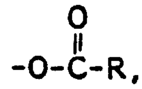, 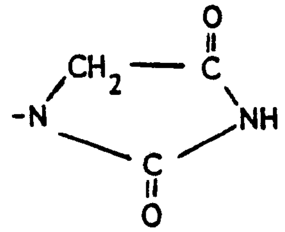, 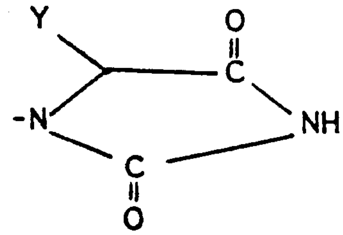

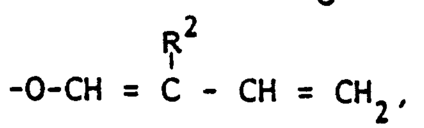, 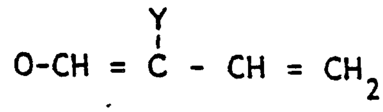

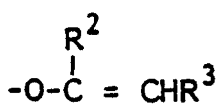 and 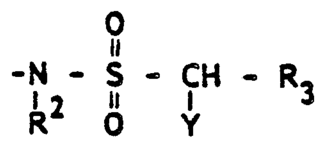,

wherein R is

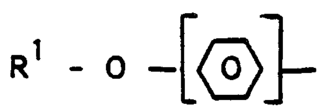

$R^1$ is as defined above, $R^2$ is an alkyl or alkylene chain containing from 1 to 8 carbon atoms, $R^3$ is H or $R^2$, and Y is H or a solubilizing group. The preferred solubilizing groups are $-SO_3^-M^+$, $-COO^-M^+$, $-SO_4^-M^+$, $(-N^+R_3^4)X^-$ and $O \leftarrow N(R_2^4)-$ and most preferably $-SO_3^-M^+$ and $-COO^-M^+$ wherein $R^4$ is an alkyl chain containing from 1 to 4 carbon atoms, M is a cation which provides solubility to the bleach activator and X is an anion which provides solubility to the bleach activator. Preferably, M is an alkali metal, ammonium or substituted ammonium cation, with sodium and potassium being most preferred, and X is a halide, hydroxide, methylsulfate or acetate anion. It should be noted that bleach activators with a leaving group that does not contain a solubilizing group should be well dispersed in the bleaching solution in order to assist in their dissolution.

Preferred bleach activators are also those of the above general formula wherein L is as defined in the general formula and $R^1$ is an alkyl group containing 6 carbon atoms.

Particularly preferred bleach activators are those of the above general formula wherein $R^1$ is an alkyl group containing from 4 to 8 carbon atoms and L is selected from the group consisting of:

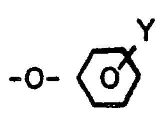, 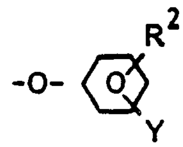 and 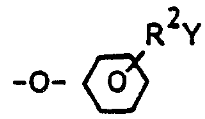

wherein $R^2$ is as defined above and Y is $-SO_3^-M^+$ or $-COO^-M^+$ wherein M is as defined above.

More preferred bleach activators are those of the above general formula wherein $R^1$ is a linear alkyl chain containing from 5 to 8, and most preferably 6 carbon atoms, and L is selected from the group consisting of:

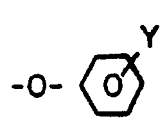, 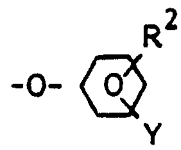 and 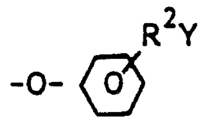

wherein $R^2$ is as defined above and Y is $-SO_3^-M^+$ or $-COO^-M^+$ wherein M is as defined above.

The most preferred bleach activators have the formula:

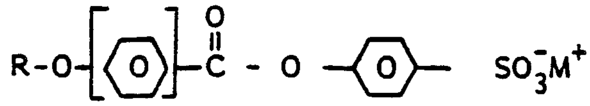

wherein R is a linear alkyl chain containing 6 carbon atoms and M is sodium or potassium.

Optimum bleaching performance is obtained with bleaching solutions wherein the pH of such solution is between 8.5 and 10.5 and preferably between 9 and 10. It is preferred that such pH be greater than 9 to optimize the rate of perhydrolysis. Such pH can be obtained with substances commonly known as buffering agents, which are optional components of the bleaching compositions herein.

The bleaching compositions of the invention, upon dissolution in aqueous solution, provide a bleaching compound of the formula

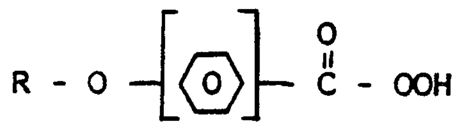

where R is an alkyl group with from 4 to 8 carbon atoms, preferably 5 to 8 carbon atoms, and most preferably 6 carbon atoms.

Such compositions provide extremely effective and efficient surface bleaching of textiles which thereby remove stains and/or soils from the textiles. The compositions are particularly effective at removing dingy soils from textiles. Dingy soils are soils that build up on textiles after numerous cycles of usage and washing, and thus, result in a white textile having a gray or yellow tint. These soils tend to be blend of particulate and greasy materials. The removal of this type of soil is sometimes referred to as "dingy fabric clean up".

The bleaching compositions provide such bleaching over a wide range of bleach solution temperatures. Such bleaching is obtained in bleach solutions wherein the solution temperature is at least 5°C. Inorganic peroxygen bleaches would be ineffective and/or impractical at temperatures below 60°C.

This invention thus also relates to bleaching compositions containing a peroxygen bleach capable of releasing hydrogen peroxide in an aqueous solution and bleach activators of the formula

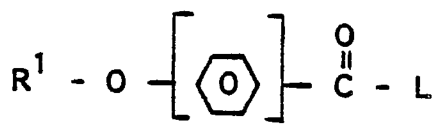

wherein $R^1$ is an alkyl group containing from 4 to 8, preferably 5 to 8, and most preferably 6 carbon atoms, and L is as defined hereinabove.

The bleaching mechanism generally, and the surface bleaching mechanism in particular, are not completely understood. However, it is generally believed that the bleach activator undergoes nucleophilic attack by a herhydroxide anion, which is generated from the hydrogen peroxide evolved by the peroxygen bleach, to form a peroxycarboxylic acid. This reaction is commonly referred to as perhydrolysis.

A second less desirable reaction involves the formation of the less reactive diacyl peroxide. This reaction increases in consequence with the hydrophobicity of R. While some diacyl peroxide production is desirable in order to improve bleaching of specific stains (e.g., spaghetti sauce or barbecue sauce) because of the lessened reactivity, conditions are preferred which produce only a small percentage of the diacyl peroxide.

It is also believed, that the bleach activators within the invention can render peroxygen bleaches more efficient even at bleach solution temperatures wherein bleach activators are not necessary to activate the bleach, i.e., above 60°C. Therefore, with bleach compositions of the invention, less peroxygen bleach is required to get the same level of surface bleaching performance as is obtained with the peroxygen bleach alone.

The bleaching compositions wherein the bleach activator is used also have, as an essential component a peroxygen bleach capable of releasing hydrogen peroxide in aqueous solution.

These compounds are well known in the art and include hydrogen peroxide and the alkali metal peroxides, organic peroxide bleaching compounds such as urea peroxide, and inorganic persalt bleaching compounds, such as the alkali metal perborates, percarbonates, perphosphates, and the like. Mixtures of two or more such bleaching compounds can also be used, if desired.

Preferred peroxygen bleaching compounds include sodium perborate, commercially available in the form of mono-, tri- and tetra- hydrates, sodium carbonate peroxyhydrate, sodium pyrophosphate peroxyhydrate, urea peroxyhydrate, and sodium peroxide. Particularly preferred are sodium perborate tetrahydrate and, especially, sodium perborate monohydrate. Sodium perborate monohydrate is especially preferred because it is very stable during storage and yet still dissolves very quickly in the bleaching solution. It is believed that such rapid dissolution results in the formation of higher levels of percarboxylic acid and, thus, enhanced surface bleaching performance.

The level by weight of peroxygen bleach within compositions of the invention is preferably from 0.1% to 95% and more preferably from 1% to 60%. When the bleaching compositions within the invention are also detergent compositions it is preferred that the level of peroxygen bleach is from 1% to 20%.

The level by weight of bleach activator within the compositions of the invention is preferably from 0.1% to 60% and more preferably from 0.5% to 40%. When the bleaching compositions within the

invention are also detergent compositions it is preferable that the level of bleach activator is from 0.5% to 20%, more preferably from 2% to 8%.

As a preferred embodiment, the bleaching compositions of the invention can be detergent compositions. Thus, the bleaching compositions can contain typical detergent composition components such as detergency surfactants and detergency builders. In such preferred embodiments the bleaching compositions are particularly effective. The bleaching compositions of this invention can contain all of the usual components of detergent compositions including the ingredients set forth in US—A—3,936,537, Baskerville et al. Such components include color speckles, suds boosters, suds suppressors, antitarnish and/or anticorrosion agents, soil-suspending agents, soil-release agents, dyes, fillers, optical brighteners, germicides, alkalinity sources, hydrotropes, antioxidants, enzymes, enzyme stabilizing agents, perfumes, etc.

The detergent surfactants can be any one or more surface active agents selected from anionic, nonionic, zwitterionic, amphoteric and cationic classes and compatible mixtures thereof. Detergent surfactants useful herein are listed in US—A—3,664,961, Norris, issued May 23, 1972, and in US—A—3,919,678, Laughlin et al, issued December 30, 1975. Useful cationic surfactants also include those described in US—A—4,222,905, Cockrell, issued September 16, 1980, and in US—A—4,239,659, Murphy, issued December 16, 1980. The following are representative examples of detergent surfactants useful in the present compositions.

Water-soluble salts of the higher fatty acids, i.e., "soaps", are useful anionic surfactants in the compositions herein. This includes alkali metal soaps such as the sodium, potassium, ammonium, and alkanolammonium salts of higher fatty acids containing from 8 to 24 carbon atoms, and preferably from 12 to 18 carbon atoms. Soaps can be made by direct saponification of fats and oils or by the neutralization of free fatty acids. Particularly useful are the sodium and potassium salts of the mixtures of fatty acids derived from coconut oil and tallow, i.e., sodium or potassium tallow and coconut soap.

Useful anionic surfactants also include the water-soluble salts, preferably the alkali metal, ammonium and alkylolammonium salts, of organic sulfuric reaction products having in their molecular structure an alkyl group containing from 10 to 20 carbon atoms and a sulfonic acid or sulfuric acid ester group. (Included in the term "alkyl" is the alkyl portion of acyl groups.) Examples of this group of synthetic surfactants are the sodium and potassium alkyl sulfates, especially those obtained by sulfating the higher alcohols ($C_8$—$C_{18}$ carbon atoms) such as those produced by reducing the glycerides of tallow or coconut oil; and the sodium and potassium alkylbenzene sulfonates in which the alkyl group contains from 9 to 15 carbon atoms, in straight chain or branched chain configuration, e.g., those of the type described in US—A—2,220,099 and US—A—2,477,383. Especially valuable are linear straight chain alkylbenzene sulfonates in which the average number of carbon atoms in the alkyl group is from 11 to 13, abbreviated as $C_{11-13}$LAS.

Other anionic surfactants herein are the sodium alkyl glyceryl ether sulfonates, especially those ethers of higher alcohols derived from tallow and coconut oil; sodium coconut oil fatty acid monoglyceride sulfonates and sulfates; sodium or potassium salts of alkyl phenol ethylene oxide ether sulfates containing from 1 to 10 units of ethylene oxide per molecule and wherein the alkyl groups contain from 8 to 12 carbon atoms; and sodium or potassium salts of alkyl ethylene oxide ether sulfates containing from 1 to 10 units of ethylene oxide per molecule and wherein the alkyl group contains from 10 to 20 carbon atoms.

Other useful anionic surfactants herein include the water-soluble salts of esters of alpha-sulfonated fatty acids containing from 6 to 20 carbon atoms in the fatty acid group and from 1 to 10 carbon atoms in the ester group; water-soluble salts of 2-acyloxyalkane-1-sulfonic acids containing from 2 to 9 carbon atoms in the acyl group and from 9 to 23 carbon atoms in the alkane moiety; water-soluble salts of olefin and paraffin sulfonates containing from 12 to 20 carbon atoms; and beta-alkyloxy alkane sulfonates containing from 1 to 3 carbon atoms in the alkyl group and from 8 to 20 carbon atoms in the alkane moiety.

Water-soluble nonionic surfactants are also useful in the compositions of the invention. Such nonionic materials include compounds produced by the condensation of alkylene oxide groups (hydrophilic in nature) with an organic hydrophobic compound, which may be aliphatic or alkyl aromatic in nature. The length of the polyoxyalkylene group which is condensed with any particular hydrophobic group can be readily adjusted to yield a water-soluble compound having the desired degree of balance between hydrophilic and hydrophobic elements.

Suitable nonionic surfactants include the polyethylene oxide condensates of alkyl phenols, e.g., the condensation products of alkyl phenols having an alkyl group containing from 6 to 15 carbon atoms, in either a straight chain or branched chain configuration, with from 3 to 12 moles of ethylene oxide per mole of alkyl phenol.

Preferred nonionics are the water-soluble and water-dispersible condensation products of aliphatic alcohols containing from 8 to 22 carbon atoms, in either straight chain or branched configuration, with from 3 to 12 moles of ethylene oxide per mole of alcohol. Particularly preferred are the condensation products of alcohols having an alkyl group containing from 9 to 15 carbon atoms with from 4 to 8 moles of ethylene oxide per mole of alcohol.

Semi-polar nonionic surfactants include water-soluble amine oxides containing one alkyl moiety of from 10 to 18 carbon atoms and two moieties selected from the group of alkyl and hydroxyalkyl moieties of from 1 to 3 carbon atoms; water-soluble phosphine oxides containing one alkyl moiety of 10 to 18 carbon

atoms and two moieties selected from the group consisting of alkyl groups and hydroxyalkyl groups containing from 1 to 3 carbon atoms; and water-soluble sulfoxides containing one alkyl moiety of from 10 to 18 carbon atoms and a moiety selected from the group consisting of alkyl and hydroxyalkyl moieties of from 1 to 3 carbon atoms.

Ampholytic surfactants include derivatives of aliphatic or aliphatic derivatives of heterocyclic secondary and tertiary amines in which the aliphatic moiety can be straight chain or branched and wherein one of the aliphatic substituents contains from 8 to 18 carbon atoms and at least one aliphatic substituent contains an anionic water-solubilizing group.

Zwitterionic surfactants include derivatives of aliphatic, quaternary, ammonium, phosphonium, and sulfonium compounds in which one of the aliphatic substituents contains from 8 to 18 carbon atoms.

The level of detergent surfactant that can be employed is from 0% to 50%, preferably from 1% to 30% and most preferably from 10% to 25% by weight of the total composition.

In addition to detergent surfactants, detergency builders can be employed in the bleaching compositions. Water-soluble inorganic or organic electrolytes are suitable builders. The builder can also be water-insoluble calcium ion exchange materials; non-limiting examples of suitable water-soluble, inorganic detergent builders include: alkali metal carbonates, borates, phosphates, bicarbonates and silicates. Specific examples of such salts include sodium and potassium tetraborates, bicarbonates, carbonates, orthophosphates, pyrophosphates, tripolyphosphates and meta-phosphates.

Examples of suitable organic alkaline detergency builders include: (1) water-soluble amino carboxylates and aminopolyacetates, for example, nitrolotriacetates, glycinates, ethylenediamine tetra-acetates, N-(2-hydroxyethyl)nitrilo diacetates and diethylenetriamine pentaacetates; (2) water-soluble salts of phytic acid, for example, sodium and potassium phytates; (3) water-soluble polyphosphonates, including sodium, potassium, and lithium salts of ethane-1-hydroxy-1, 1-diphosphonic acid; sodium, potassium, and lithium salts of ethylene diphosphonic acid; and the like; (4) water-soluble polycarboxylates such as the salts of lactic acid, succinic acid, malonic acid, maleic acid, citric acid, carboxy-methyloxysuccinic acid, 2-oxa-1,1,3-propane tricarboxylic acid, 1,1,2,2-ethane tetracarboxylic acid, mellitic acid and pyromellitic acid; and (5) water-soluble polyacetals as disclosed in US—A—4,144,266 and US—A—4,246,495.

Another type of detergency builder material useful in the present compositions comprises a water-soluble material capable of forming a water-insoluble reaction product with water hardness cations preferably in combination with a crystallization seed which is capable of providing growth sites for said reaction product. Such "seeded builder" compositions are fully disclosed in GB—A—1,424,406.

A further class of detergency builder materials useful in the present invention are insoluble sodium aluminosilicates, particularly those described in BE—A—814,874, issued November 12, 1974. This patent discloses and claims detergent compositions containing sodium aluminosilicates having the formula:

$$Na_z(AlO_2)_z(SiO_2)_yXH_2O$$

wherein z and y are integers equal to at least 6, the molar ratio of z to y is in the range of from 1.0:1 to 0.5:1, and X is an integer from 15 to 264, said aluminosilicates having a calcium ion exchange capacity of at least 200 milligrams equivalent/gram and a calcium ion exchange rate of at least 130 mg equivalent of $CaCO_3$/litre/minute/gram [2 grains/gallon/minute/gram]. A preferred material is Zeolite A which is:

$$Na_{12}(SiO_2AlO_2)_{12}27H_2O \quad .$$

The level of detergency builder of the bleaching compositions is from 0% to 70%, preferably from 10% to 60% and most preferably from 20% to 60%.

Buffering agents can be utilized to maintain the desired alkaline pH of the bleaching solutions. Buffering agents include, but are not limited to many of the detergency builder compounds disclosed hereinbefore. Buffering agents suitable for use herein are those well known in the detergency art.

Preferred optional ingredients include suds modifiers particularly those of suds suppressing types, exemplified by silicones, and silica-silicone mixtures.

US—A—3,933,672, issued January 20, 1976 to Bartolotta et al, and US—A—4,136,045, issued January 23, 1979 to Gault et al, disclose silicone suds controlling agents. The silicone material can be represented by alkylated polysiloxane materials such as silica aerogels and xerogels and hydrophobic silicas of various types. The silicone material can be described as siloxane having the formula:

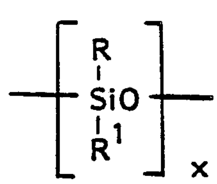

wherein x is from 20 to 2,000 and R and $R^1$ are each alkyl or aryl groups, especially methyl, ethyl, propyl, butyl and phenyl. The polydimethylsiloxanes (R and $R^1$ are methyl) having a molecular weight within the

range of from 200 to 2,000,000, and higher, are all useful as suds controlling agents. Additional suitable silicone materials wherein the side chain groups R and $R^1$ are alkyl, aryl, or mixed alkyl or aryl hydrocarbyl groups exhibit useful suds controlling properties. Examples of the like ingredients include diethyl-, dipropyl-, dibutyl-, methyl-, ethyl-, phenylmethylpoly-siloxanes and the like. Additional useful silicone suds controlling agents can be represented by a mixture of an alkylated siloxane, as referred to hereinbefore, and solid silica. Such mixtures are prepared by affixing the silicone to the surface of the solid silica. A preferred silicone suds controlling agent is represented by a hydrophobic silanated (most preferably trimethylsilanated) silica having a particle size in the range from 10 nm to 20 nm and a specific surface area above 50 $m^2$/gm. intimately admixed with dimethyl silicone fluid having a molecular weight in the range from 500 to 200,000 at a weight ratio of silicone to silanated silica of from 19:1 to 1:2. The silicone suds suppressing agent is advantageously releasable incorporated in a water-soluble or water-dispersible, substantially non-surface-active detergent-impermeable carrier.

Particularly useful suds suppressors are the self-emulsifying silicone suds suppressors, described in US—A—4,073,118, Gault et al, issued February 21, 1978. An example of such a compound is DB—544, commercially available from Dow Corning, which is a siloxane/glycol copolymer.

Suds modifiers as described above are used at levels of up to approximately 2%, preferably from 0.1 to 1½% by weight of the surfactant.

Microcrystalline waxes having a melting point in the range from 35°C—115°C and a saponification value of less than 100 represent additional examples of preferred suds control components for use in the subject compositions, and are described in detail in US—A—4,056,481, Tate, issued November 1, 1977. The microcrystalline waxes are substantially water-insoluble, but are water-dispersible in the presence of organic surfactants. Preferred microcrystalline waxes have a melting point from 65°C to 100°C, a molecular weight in the range from 400—1,000; and a penetration value of at least 6, measured at 25°C by ASTM—D1321. Suitable examples of the above waxes include: microcrystalline and oxidized microcrystalline petroleum waxes; Fischer-Tropsch and oxidized Fischer-Tropsch waxes; ozokerite; ceresin; montan wax; beeswax; candelilla; and carnbauba wax.

Alkyl phosphate esters represent an additional preferred suds control agent for use herein. These preferred phosphate esters are predominantly monostearyl phosphate which, in addition thereto, can contain di- and tristearyl phosphates and monooleyl phosphate, which can contain di- and trioleyl phosphate.

Other suds control agents useful in the practice of the invention are the soap or the soap and nonionic mixtures as disclosed in US—A—2,954,347 and US—A—2,954,348.

The following examples are given to illustrate the parameters of and compositions within the invention. All percentages, parts and ratios are by weight unless otherwise indicated.

## Example I

Preparation of sodium 4-hexyloxybenzoyloxybenzenesulfonate.

The anhydrous disodium salt of p-phenolsulfonic acid was prepared as follows. To a slurry of sodium p-phenolsulfonic acid dihydrate (250 g, 1.08 mol) in 200 mL distilled water was added 43.0 g (1.08 mol) of sodium hydroxide as a 50.5% solution. The resulting slurry was evaporated on a rotary evaporator to yield a hard, white solid. This solid was dried to constant weight in a convection oven at 150°C to yield 229 g (97%) of the anhydrous disodium salt. To a suspension of 45.2 g (0.207 mol) of the above anhydrous disodium salt of p-phenolsulfonic acid, in 250 mL ethylene glycol dimethyl ether was added in one portion a solution of 50.0 g (0.207 mol) of 4-hexyloxybenzoyl chloride in 50 mL ethylene glycol dimethyl ether. The resulting stirred suspension was refluxed under nitrogen for 3 hours, cooled, filtered, and the collected solid washed with diethyl ether. The resulting solid was stirred with 200 mL water, filtered, and washed with water. The solid was then stirred with 150 mL methanol, filtered, and washed with methanol. Vacuum drying afforded 64.2 g (78%) of sodium 4-hexyloxybenzoyloxybenzenesulfonate as an off-white solid.

## Example II

Preparation of sodium 4-butoxybenzoyloxybenzenesulfonate.

The general procedure described in Example 1 was followed. Anhydrous p-phenolsulfonic acid, disodium salt (51.2 g., 0.235 mol), 4-butoxybenzoyl chloride (50.0 g, 0.235 mol), and 250 mL ethylene glycol dimethyl ether were refluxed under nitrogen for 4 hours. The resulting mixture was cooled in an ice bath, filtered, and the collected solid washed with diethyl ether. The crude solid (85.6 g) was stirred with 150 mL ice-cold water, filtered, and washed with ice-cold water. While still on the filter the solid was washed with three 25 mL portions of ice-cold methanol. Yield (after vacuum drying) was 56.0 g of white solid. The NMR spectrum (dimethyl-$d_6$ sulfoxide solvent) showed that the solid consisted of 95% sodium 4-butoxybenzoyloxybenzenesulfonate and 5% sodium p-phenolsulfonate. The yield of purified sodium 4-butoxybenzoyloxybenzenesulfonate was 61%.

## Example III

Preperation of blend of sodium 4-hexyloxybenzoyloxybenzenesulfonate with sodium sulfate and sodium linear alkylbenzene sulfonate (LAS).

A weight of 11.8 g of 85% active $C_{11.6}$ LAS was dissolved in 50 mL water and the pH adjusted to 7.0 with 0.1 N sulfuric acid. This solution was blended with 40.0 g of sodium 4-hexyloxybenzoyloxybenzenesulfonate (powdered and passed through 0.707 mm sieve (24 mesh)) and 100.0 g of powdered sodium sulfate. The resulting paste was then dried under vacuum, powdered, and passed through 0.707 mm sieve to yield a rapidly soluble composition containing 26.4% sodium 4-hexyloxybenzoyloxybenzenesulfonate.

Example IV

Perhydrolysis of sodium 4-hexyloxybenzoyloxybenzenesulfonate to form 4-hexyloxyperoxybenzoic acid.

A detergent granule having the following composition was prepared:

| | % |
|---|---|
| Sodium $C_{13}$ linear alkylbenzene sulfonate | 8.5 |
| Sodium $C_{14-15}$ alkyl sulfate | 8.5 |
| $C_{12-13}$ alkyl polyethoxylate$_{6.5}$ | 2.3 |
| $C_{12}$ alkyl trimethylammonium chloride | 1.1 |
| Sodium tripolyphosphate | 36.4 |
| | % |
| Sodium carbonate | 13.6 |
| Sodium silicate | 5.7 |
| Sodium sulfate | 11.7 |
| Moisture | 8.5 |
| Minor ingredients | 3.6 |

A 4 liter flask was equipped with a magnetic stirrer and charged with 4 L of 35°C (95°F) city water, 5.00 g of the above detergent granules, 0.21 g of sodium perborate monohydrate, and 0.89 g of the composition described in Example III. The resulting solution was stirred, aliquots withdrawn at various time intervals, and the aliquots analyzed for organic peroxyacid by a conventional iodometric method. This method consisted of pouring the solution aliquot onto ice, acidification of the resulting mixture with acetic acid, addition of potassium iodide solution, and titration of the liberated iodine with sodium thiosulfate solution. The volume of sodium thiosulfate solution required was then used to calculate the concentration in solution of available oxygen (AvO) due to the formation of organic peroxyacid. The concentration of AvO was then used to calculate the concentration of 4-hexyloxyperoxybenzoic acid.

Tabulated below are the results obtained for the above experiment.

| | | 4-hexyloxyperoxybenzoic acid | |
|---|---|---|---|
| Time (min.) | [Peroxyacid AvO] (ppm) | Concentration in ppm | % of theoretical yield |
| 2 | 0.56 | 8.35 | 23 |
| 7 | 1.17 | 17.4 | 49 |
| 12 | 1.37 | 20.4 | 57 |
| 20 | 1.60 | 23.8 | 67 |

Example V

Bleaching performance of 4-hexyloxyperoxybenzoic acid.

In this Example 4-hexyloxyperoxybenzoic acid was formed during the washing cycle by the reaction of sodium 4-hexyloxybenzoyloxybenzene sulfonate with hydrogen peroxide (from sodium perborate monohydrate) in the presence of the detergent granules described in Example IV. Bleaching performance was determined by comparing the fabric whitening and stain removal of this treatment with a treatment containing only the detergent granule. Thus, to each of two top-loading automatic washing machines was added 2.27 kg (5 lbs) of naturally soiled ballast fabrics and 68 liters of 35°C (95°F) city water having a

hardness of 102.6 mg equivalent $CaCO_3$/litre (6 gr/gal). To one machine was added 89 g of the detergent granules described in Example IV, and to a second machine was added 89 g of this same detergent granule, 6.1 g of sodium perborate monohydrate, and 27.0 g of a composition like that described in Example III and containing 22/7% of sodium 4-hexyloxybenzoyloxybenzene sulfonate. The wash solution in this second machine thus contained concentrations of 90 ppm of both sodium perborate monohydrate and sodium 4-hexyloxybenzoyloxybenzene sulfonate.

To each of the above wash solutions were added two sets of naturally soiled white fabrics and two sets of artificially stained swatches. The washing machines were then allowed to complete their normal washing and rinsing cycles, and the ballast and test fabrics were dryer dried. This procedure was repeated three times, using different sets of ballast fabrics, naturally soiled white fabrics and artificially stained swatches for each replicate.

After completion of the three replicates, the fabrics and swatches were arranged under suitable lighting for comparison of soil and stain removal. Three expert graders compared the extent of removal of the soils and stains using the following scale:

0 no difference between two swatches
1 thought to be a difference
2 certain of a difference
3 certain of a large difference
4 certain of a very large difference

In this grading the naturally soiled white fabrics were compared for improvement in whiteness, and the artificially stained swatches were compared for removal of the stain. The grades obtained were then averaged and normalized to yield the results shown below. Also tabulated below are the percentage of grades for each fabric or swatch type where the treatment containing sodium 4-hexyloxybenzoyloxybenzene sulfonate and sodium perborate monohydrate was superior to the detergent alone treatment by a score of 2 or more on the above scale.

| | Treatment and Average Relative Grade | |
|---|---|---|
| | Detergent Granule Alone | Detergent granule + sodium 4-hexyloxybenzoyloxybenzene sulfonate + sodium perborate monohydrate |
| Naturally Soiled Fabrics | | |
| T-shirts | 0 | 2.0 |
| Dish towels | 0 | 1.6 |
| Pillowcases | 0 | 1.3 |
| Artificially Stained Fabrics | | |
| Clay | 0 | 0.9 |
| Spaghetti sauce | 0 | 2.0 |
| Barbecue sauce | 0 | 0.2 |
| Tea | 0 | 1.7 |
| Grass | 0 | 1.8 |
| Animal blood | 0 | -0.1 |
| Blueberries | 0 | 1.2 |

| | % of Grades ≧ 2 Detergent granule + sodium 4-hexyloxybenzoyloxybenzene sulfonate + sodium perborate monohydrate versus detergent granule alone |
|---|---|
| **Naturally Soiled Fabrics** | |
| T-shirts | 89 |
| Dish towels | 56 |
| Pillowcases | 39 |
| **Artificially Stained Fabrics** | |
| Clay | 50 |
| Spaghetti sauce | 83 |
| Barbecue sauce | 44 |
| Tea | 65 |
| Grass | 72 |
| Animal blood | 17 |
| Blueberries | 44 |

Example VI

Performance of 4-but-, 4-pentyl-, and 4-hexyl-oxyperoxybenzoic acids in the presence of both clean and soiled ballast loads.

4-but-, 4-pentyl-, and 4-hexyl-oxyperoxy-benzoic acids were formed by the reactions of the corresponding sodium alkyloxybenzoyloxybenzene sulfonates with hydrogen peroxide from sodium perborate monohydrate in the presence of the detergent granule described in Example IV. Measurements of bleaching performance were conducted with 7.6 liter solutions in 35°C (95°F) water containing 85.5 mg equivalent $CaCO_3$/litre (5 gr/gal) hardness as 3:1 molar $Ca^{+2}$:$Mg^{+2}$. The performance of solutions containing 1250 ppm of the detergent granule, 90 ppm of sodium perborate monohydrate, and 90 ppm of a sodium 4-alkyloxybenzoyloxybenzene sulfonate were compared with a solution containing 1250 ppm of the detergent granule alone. These comparisons were made also where the ballast load in each solution was 200 g of clean terrycloth and also where the ballast load consisted of 100 g of naturally soiled T-shirt material plus 100 g of naturally soiled pillowcases. The soiled fabrics and stained swatches were the same as employed in Example V. The washed fabrics and swatches were graded in a round robin fashion and the relative performance of the various treatments was calculated by standard methods. The results obtained are tabulated below. These results demonstrate that the bleaching performance of the 4-alkyloxyperoxybenzoic acids is relatively unaffected by the presence of soil.

Test 1

Bleaching Performance With a Clean, Terrycloth Ballast

| | Concentration (ppm) | | | |
|---|---|---|---|---|
| Detergent granule | 1250 | 1250 | 1250 | 1250 |
| Sodium perborate monohydrate | | 90 | 90 | 90 |
| Sodium 4-butoxybenzoyloxy-benzene sulfonate | | 90 | | |
| Sodium 4-pentyloxybenzoyloxy-benzene sulfonate | | | 90 | |
| Sodium 4-hexyloxybenzoyloxy-benzene sulfonate | | | | 90 |
| Naturally Soiled Fabrics | Relative Grades | | | |
| T-shirts | 0 | 2.0 | 2.4 | 2.1 |
| Dish towels | 0 | 1.5 | 0.9 | 1.1 |
| Pillowcases | 0 | 1.2 | 1.6 | 1.6 |
| Artificially Stained Swatches | | | | |
| Clay | 0 | -0.2 | 0.1 | 0.3 |
| Spaghetti sauce | 0 | 2.3 | 2.1 | 1.6 |
| Barbecue sauce | 0 | 1.9 | 1.3 | 1.3 |
| Tea | 0 | 2.4 | 2.1 | 1.9 |
| Grass | 0 | 1.4 | 2.1 | 2.8 |
| Animal blood | 0 | 0.0 | 0.9 | 1.0 |
| Blueberries | 0 | 2.3 | 1.5 | 1.3 |

Test 2

Bleaching Performance With a Soiled Ballast of T-Shirts and Pillowcases

| | Concentration (ppm) | | | |
|---|---|---|---|---|
| Detergent granule | 1250 | 1250 | 1250 | 1250 |
| Sodium perborate monohydrate | | 90 | 90 | 90 |
| Sodium 4-butoxybenzoyloxybenzene sulfonate | | 90 | | |
| Sodium 4-pentyloxybenzoyloxybenzene sulfonate | | | 90 | |
| Sodium 4-hexyloxybenzoyloxybenzene sulfonate | | | | 90 |
| | Relative Grades | | | |
| Naturally Soiled Fabrics | | | | |
| T-shirts | 0 | 2.1 | 1.4 | 2.3 |
| Dish towels | 0 | 2.0 | 1.9 | 1.9 |
| Pillowcases | 0 | 1.8 | 1.6 | 1.3 |
| Artificially Stained Swatches | | | | |
| Clay | 0 | -0.5 | -0.4 | -0.1 |
| Spaghetti sauce | 0 | 2.7 | 2.2 | 1.4 |
| Barbecue sauce | 0 | 2.2 | 1.9 | 1.6 |
| Tea | 0 | 2.4 | 1.8 | 2.1 |
| Grass | 0 | 1.3 | 2.3 | 2.2 |
| Animal blood | 0 | 1.3 | 1.4 | 1.4 |
| Blueberries | 0 | 2.2 | 1.9 | 1.4 |

Example VII

Bleaching Performance of 4-Hexyloxyperoxybenzoic Acid

The data contained in this Example were obtained using the procedures described in Example V. The treatment containing 4-hexyloxyperoxybenzoic acid consisted of 89 g of the detergent granule described in Example IV, 3.6 g of sodium perborate monohydrate, 15.3 g of a composition like that of Example III and containing 26.7% of sodium 4-hexyloxybenzoyloxybenzene sulfonate, and 0.6 g of an enzyme composition containing protease and having an activity of 2.2 Anson units/g. The resulting wash solution thus contained 52.5 ppm of sodium perborate monohydrate and 60 ppm of sodium 4-hexyloxybenzoyloxybenzene sulfonate. The bleaching performance of this treatment was compared with a treatment that contained 89 g of the base granule alone. The results obtained are tabulated below.

The odors of both the wash solutions and the wet fabrics from the treatments that contained 4-hexyloxyperoxybenzoic acid were evaluated by an expert perfumer; this evaluation indicated a complete absence of the malodors that are frequently associated with the use of peroxyacid bleaches.

| | Treatment and Average Relative Grade | |
|---|---|---|
| | Detergent Granule Alone | Detergent granule + sodium 4-hexyloxy-benzoyloxybenzene sulfonate + sodium perborate monohydrate + enzymes |
| **Naturally Soiled Fabrics** | | |
| T-shirts | 0 | 1.6 |
| Dish towels | 0 | 1.3 |
| Pillowcases | 0 | 1.4 |
| **Artificially Stained Fabrics** | | |
| Clay | 0 | 1.2 |
| Spaghetti sauce | 0 | 1.9 |
| Barbecue sauce | 0 | 1.3 |
| Tea | 0 | 2.2 |
| Grass | 0 | 3.2 |
| Animal blood | 0 | 0.2 |
| Blueberries | 0 | 2.2 |

| | % of Grades ≥ 2 |
|---|---|
| | Detergent granule + sodium 4-hexyloxy-benzoyloxybenzene sulfonate + sodium perborate monohydrate + enzymes versus detergent granule alone |
| **Naturally Soiled Fabrics** | |
| T-shirts | 67 |
| Dish towels | 44 |
| Pillowcases | 44 |
| **Artificially Stained Fabrics** | |
| Clay | 67 |
| Spaghetti sauce | 78 |
| Barbecue sauce | 61 |
| Tea | 89 |
| Grass | 100 |
| Animal blood | 28 |
| Blueberries | 100 |

**Claims**

1. A bleach activator of the general formula

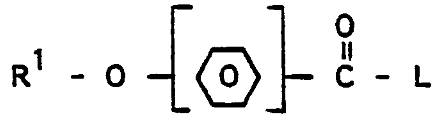

wherein $R^1$ is an alkyl group containing from 4 to 8 carbon atoms, and L is a leaving group, the conjugate acid of which has a $pK_a$ in the range of from 4 to 13.

2. A compound according to Claim 1 wherein $R^1$ is an alkyl group containing from 5 to 8 carbon atoms.

3. A compound according to Claim 1 wherein $R^1$ is an alkyl group containing 6 carbon atoms.

4. A compound according to Claim 1 wherein L is a leaving group, the conjugate acid of which has a $pK_a$ in the range of from 8 to 11.

5. A compound according to Claim 1 wherein L is selected from the group consisting of:

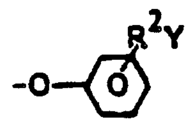 , 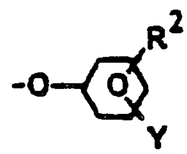 , 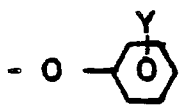 , 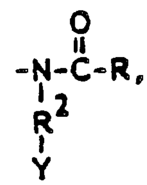

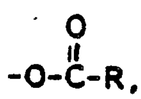 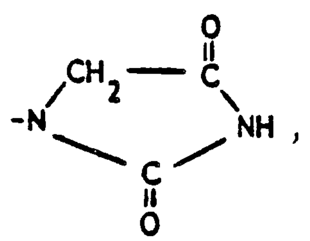 , 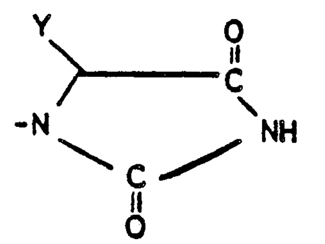 ,

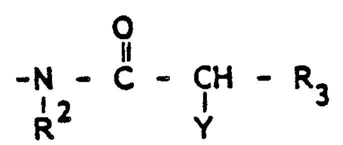 , 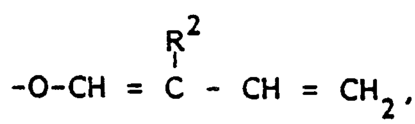

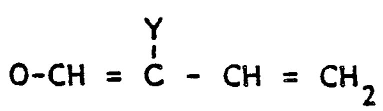 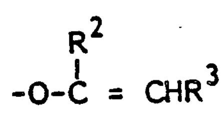 and 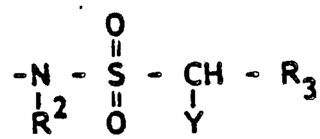 ,

wherein R is

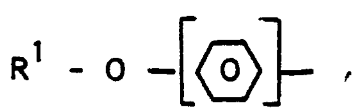 ,

$R^2$ is an alkyl or alkylene chain containing from 1 to 8 carbon atoms, $R^3$ is H or $R^2$, and Y is H or a solubilizing group.

6. A compound according to Claim 5 wherein Y is selected from the group consisting of: $—SO_3^-M^+$, $—COO^-M^+$, $—SO_4^-M^+$, $(—N^+R_3^4)X^-$ and $O \leftarrow NR_2^4$ — and mixtures thereof wherein $R^4$ is an alkyl chain containing from 1 to 4 carbon atoms, M is a cation which provides solubility to the bleach activator and X is an anion which provides solubility to the bleach activator.

7. A compound according to Claim 6 wherein Y is selected from the group consisting of $—SO_3^-M^+$, $—COO^-M^+$ and mixtures thereof wherein M is selected from the group consisting of sodium, potassium and mixtures thereof.

8. A compound according to Claim 5 wherein L is selected from the group consisting of:

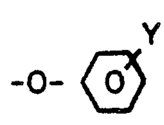 , 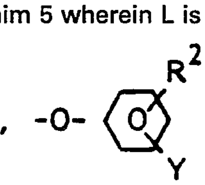 and 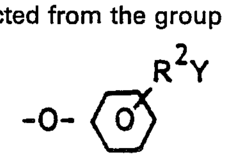

wherein $R^2$ is an alkyl or alkylene chain containing from 1 to 8 carbon atoms, Y is $-SO_3^-M^+$ or $-COO^-M^+$ wherein M is sodium or potassium.

9. A compound according to Claim 8 wherein L has the general formula:

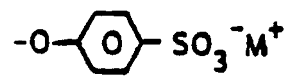

wherein M is sodium or potassium.

10. A bleaching composition comprising:

a) a peroxygen bleaching compound capable of yielding hydrogen peroxide in an aqueous solution; and

b) a bleach activator according to Claim 1, wherein the molar ratio of hydrogen peroxide yielded by (a) to bleach activator (b) is at least 1.

11. A composition according to Claim 10 wherein the peroxygen bleaching compound is selected from the group consisting of sodium perborate monohydrate, sodium perborate tetrahydrate, sodium carbonate peroxyhydrate, sodium pyrophosphate peroxyhydrate, urea peroxyhydrate, sodium peroxide and mixtures thereof.

12. A bleaching detergent composition according to claim 10 comprising, by weight:

a) from 1% to 60% of said peroxygen bleaching compound;

b) from 0.5% to 40% of said bleach activator;

wherein the molar ratio of hydrogen peroxide yielded by (a) to bleach activator (b) is greater than 1.0; and

c) from 1% to 30% of a detergent surfactant.

**Patentansprüche**

1. Ein Bleichaktivator der allgemeinen Formel

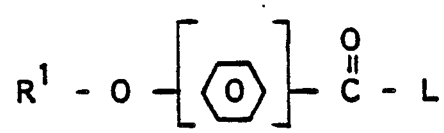

worin $R^1$ eine Alkylgruppe, enthaltend 4 bis 8 Kohlenstoffatome, bedeutet und L eine Abgangsgruppe ist, deren konjugate Säure einen pKa-Wert im Bereich von 4 bis 13 hat.

2. Eine Verbindung gemäss Anspruch 1, worin $R^1$ eine Alkylgruppe, enthaltend 5 bis 8 Kohlenstoffatome, ist.

3. Eine Verbindung gemäss Anspruch 1, worin $R^1$ eine Alkylgruppe, enthaltend 6 Kohlenstoffatome, ist.

4. Eine Verbindung gemäss Anspruch 1, worin L eine Abgangsgruppe ist, deren konjugate Säure einen pKa-Wert im Bereich von 8 bis 11 hat.

5. Eine Verbindung gemäss Anspruch 1, worin L ausgewählt ist aus der Gruppe, bestehend aus

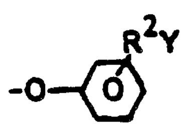, 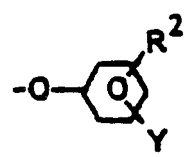, 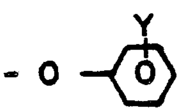, 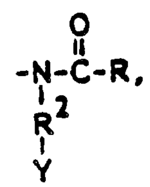

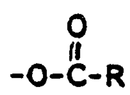, 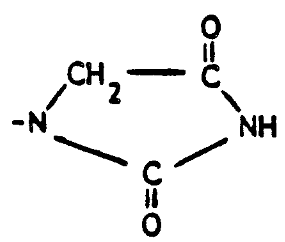, 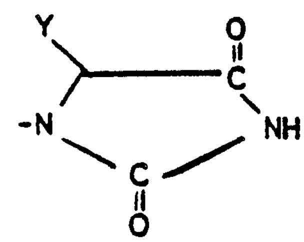,

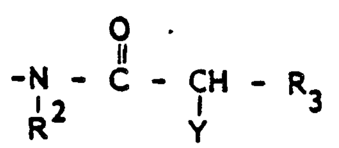, 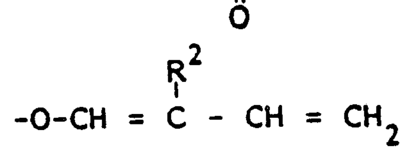,

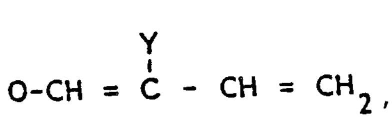, 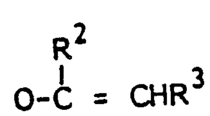 und 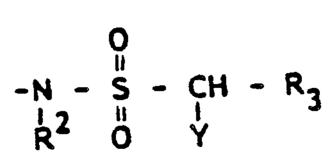,

worin R 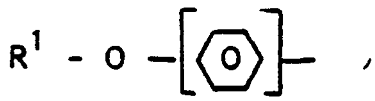

bedeutet, $R^2$ eine Alkyl- oder Alkylenkette, enthaltend 1 bis 8 Kohlenstoffatome, bedeutet, $R^3$ H oder $R^2$ ist und Y H oder eine löslichmachende Gruppe ist.

6. Eine Verbindung gemäss Anspruch 5, worin Y ausgewählt ist aus der Gruppe, bestehend aus: $—SO_3^-M^+$, $—COO^-M^+$, $SO_4^-M^+$, $(N^+R_3^4)X^-$ und O $NR_2^4$ — und deren Mischungen, worin $R^4$ eine Alkylkette, enthaltend 1 bis 4 Kohlenstoffatome, bedeutet, M ein Kation ist, welches dem Bleichaktivator Löslichkeit verschafft, und X ein Anion ist, welches dem Bleichaktivator Löslichkeit verschafft.

7. Eine Verbindung gemäss Anspruch 6, worin Y ausgewählt ist aus der Gruppe bestehend aus $—SO_3^-M^+$, $—COO^-M^+$ und deren Mischungen, worin M ausgewählt ist aus der Gruppe, bestehend aus Natrium, Kalium und deren Mischungen.

8. Eine Verbindung gemäss Anspruch 5, worin L ausgewählt ist aus der Gruppe, bestehend aus:

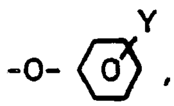 , 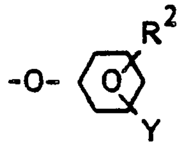 und 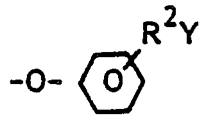

worin $R^2$ eine Alkyl- oder Alkylenkette, enthaltend 1 bis 8 Kohlenstoffatome, bedeutet, Y $—SO_3^-$ $M^+$ oder $—COO^-M^+$ ist, worin M Natrium oder Kalium ist.

9. Eine Verbindung gemäss Anspruch 8, worin L die allgemeine Formel

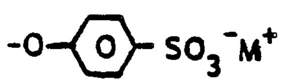

hat, worin M Natrium oder Kaliûm ist.

10. Eine Bleich-Zusammensetzung, enthaltend:

a) eine Peroxy-Bleichverbindung, die fähig ist, in wässriger Lösung Wasserstoffperoxid hervorzubringen, und

b) einen Bleichaktivator gemäss Anspruch 1, worin das molare Verhältnis von Wasserstoffperoxid, erhalten aus (a) und Bleichaktivator (b) wenigstens 1 beträgt.

11. Eine Zusammensetzung gemäss Anspruch 10, worin die Peroxy-Bleichverbindung ausgewählt ist aus der Gruppe, bestehend aus Natriumperborat-monohydrat, Natriumperborat-tetrahydrat, Natriumcarbonat-peroxyhydrat, Natriumpyrophosphat-peroxyhydrat, Harnstoff-peroxyhydrat, Natriumperoxid und deren Mischungen.

12. Eine Bleich-Detergenszusammensetzung gemäss Anspruch 10, enthaltend (Gewichtsprozent):

a) 1 bis 10% dieser Peroxy-Bleichverbindung,

b) 0,5 bis 40% dieses Bleichaktivators, wobei das molare Verhältnis von Wasserstoffperoxid, erhalten aus (a) und dem Bleichaktivator (b) grösser als 1,0 ist, und

c) 1 bis 30% eines oberflächenaktiven Detergens.

**Revendications**

1. Activateur de blanchiment de formule générale

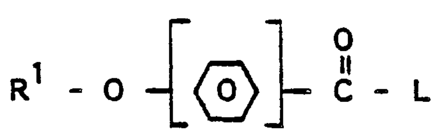

dans laquelle $R^1$ est un groupe alkyle contenant de 4 à 8 atomes de carbone et L est un groupe éliminable, dont l'acide conjugué a un $pK_a$ compris entre 4 et 13.

2. Composé selon la revendication 1, dans lequel $R^1$ est un groupe alkyle contenant de 4 à 8 atomes de carbone.

3. Composé selon la revendication 1, dans lequel $R^1$ est un groupe alkyle contenant 6 atomes de carbone.

4. Composé selon la revendication 1, dans lequel L est un groupe éliminable dont l'acide conjugué a un $pK_a$ compris entre 8 et 11.

5. Composé selon la revendication 1, dans lequel L est choisi dans le groupe comprenant:

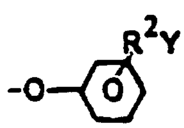 , 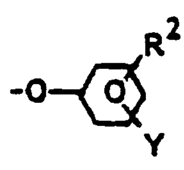 , 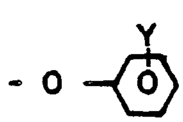 , 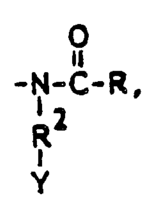

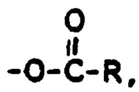, 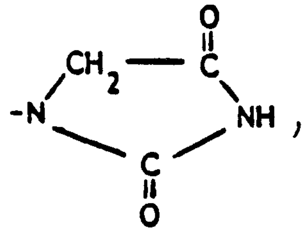, 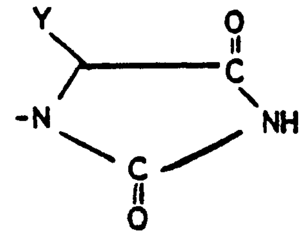,

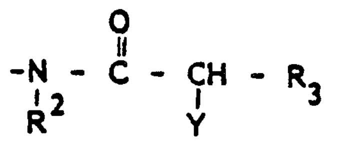, 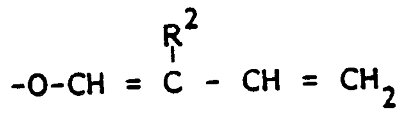,

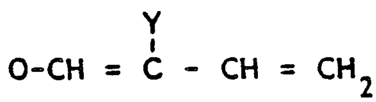, 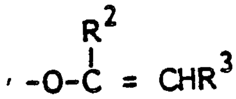 et 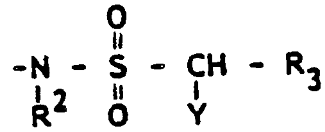,

où R est

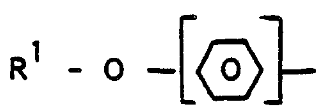,

$R^2$ est un chaîne alkyle ou alkylène contenant de 1 à 8 atomes de carbone, $R^3$ est H ou $R^2$ et Y est H ou un groupe solubilisateur.

6. Composé selon la revendication 5, dans lequel Y est choisi dans le groupe comprenant $—SO_3^-M^+$, $—COO^-M^+$, $—SO_4^-M^+$, $(—N^+R_3^4)X^-$ et $O{\leftarrow}NR_2^4$ et leurs mélanges, où $R^4$ est une chaîne alkyle contenant de 1 à 4 atomes de carbone, M est un cation conférant une propriété de solubilité à l'activateur de blanchiment, et X est un anion qui confère une propriété de solubilité à l'activateur de blanchiment.

7. Composé selon la revendication 6, dans lequel Y est choisi dans le groupe comprenant $—SO_3^-M^+$, $—COO^-M^+$ et leurs mélanges, où M est choisi dans le groupe comprenant le sodium, le potassium et leurs mélanges.

8. Composé selon la revendication 5, dans lequel L est choisi dans le groupe comprenant:

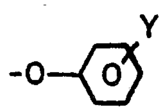, 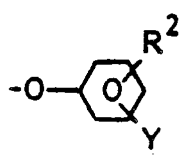 et 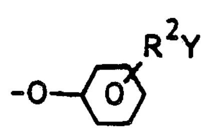

où $R^2$ est une chaîne alkyle ou alkylène contenant de 1 à 8 atomes de carbone, Y est $—SO_3^-M^+$ ou $—COO^-M^+$, où M est le sodium ou le potassium.

9. Composé selon la revendication 8, dans lequel L a la formule générale suivante:

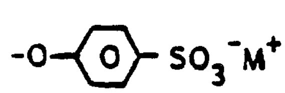

dans laquelle M est le sodium ou le potassium.

10. Composition de blanchiment comprenant:

a) un composé de blanchiment peroxygéné capable de fournir du peroxyde d'hydrogène en solution aqueuse; et

b) un activateur de blanchiment selon la revendication 1,

dans laquelle le rapport en moles entre le peroxyde d'hydrogène obtenu en (a) et l'activateur de blanchiment (b) est d'au moins 1.

11. Composition selon la revendication 10, dans laquelle le composé de blanchiment peroxygéné est choisi dans le groupe comprenant le perborate de sodium monohydraté, le perborate de sodium tétrahydraté, le carbonate de sodium peroxyhydraté, le pyrophosphate de sodium peroxyhydraté, l'urée peroxyhydratée, le peroxyde de sodium et leurs mélanges.

12. Composition détergente de blanchiment selon la revendication 10, comprenant, en poids:
a) de 1 à 60% dudit composé de blanchiment peroxygéné,
b) de 0,5 à 40% dudit activateur de blanchiment,
dans laquelle le rapport en moles entre le peroxyde d'hydrogène obtenu en (a) et l'activateur de blanchiment (b) est supérieur à 1,0; et
c) de 1 à 30% d'un tensioactif détergent.